# EUROPEAN PATENT APPLICATION

(11) **EP 4 467 972 A1**
(43) Date of publication of application: **27.11.2024**
(21) Application number: 23382489.5
(22) Date of filing: 25.05.2023
(51) Int. Cl.: G01N 27/30, G01N 27/40, G01N 27/48

(54) **SYSTEM AND METHOD FOR DETERMINING THE PRESENCE AND/OR CONCENTRATION OF NANOPARTICLES IN A SAMPLE AND USES THEREOF**

(71) Applicant: Universidad de Oviedo, 33003 Oviedo (ES)
(72) Inventor: BLANCO LÓPEZ, María del Carmen, 33006 Oviedo (ES); SÁNCHEZ CALVO, Alberto, 33006 Oviedo (ES); GUTIÉRREZ CERVELLÓ, Gemma, 33006 Oviedo (ES); MATOS GONZÁLEZ, María, 33006 Oviedo (ES); SERRANO PERTIERRA, Esther, 33006 Oviedo (ES)
(74) Representative: Hoffmann Eitle

(57) **Abstract**

A system for determining the presence and/or concentration of nanoparticles in a sample comprising a device and a detection reagent. The detection reagent comprises an electrochemical probe, and the device comprises: a) an electrochemical sensor characterized by comprising at least one working electrode and a second electrode; b) a sample application area in fluid communication with the electrochemical sensor; c) a membrane immobilized on said working electrode, the membrane being characterized by comprising nanochannels that control the sample (20) access to the working electrode; and d) means for determining the difference in the electrochemical state between the working electrode and the second electrode. In the system, the presence and/or concentration is determined from the change in the difference in the electrochemical state caused by the blockage of the nanochannels by the presence of the nanoparticles in the sample. The invention also refers to uses and methods thereof.

## Description

### TECHNICAL FIELD

The present invention relates to the field of the life sciences, including medical, veterinary, botanic, environmental, well-being, nutrition, food manipulation, in-vivo diagnostics, in-vitro diagnostics and prognosis. Particularly, the present invention relates to the field of nanoparticle sensors, more particularly to a system for determining the presence and/or concentration of nanoparticles in a sample, to its use for food, cosmetic and pharmaceutical safety control and to a method thereof.

### BACKGROUND ART

The quantification of different types of nanoparticles, whether artificially synthesised or of natural origin, is essential in various fields or applications. Some of the nanoparticles of most interest in biotechnology are gold or silver, carbon, copper, iron oxides or quantum dots (semiconducting particles with nanometre sizes that give them both optical and electrochemical properties). All of these are being explored in biosensor design.

The characterisation of nanoparticle suspensions must include their quantification, in order to control the production of different batches. In addition, quantification methods are also needed because of their potential environmental impact. Several studies have confirmed the presence of nanoparticles in the environment, whether in soil, water or air, and even in the human body. Although the possible toxicological effects of nanoparticles are not yet fully defined, this is a factor to be taken into account, and it is necessary to develop rapid and effective methods for their quantification.

On the other hand, with respect to naturally synthesised nanoparticles, extracellular vesicles stand out for their high importance in cell biology and biomedicine. These types of extracellular vesicles such as exosomes or microvesicles are used as mediators between cells for the transfer of substances necessary for the maintenance of functions in organisms. Exosomes are being explored as a source of biomarkers in various types of diseases (respiratory, cardiovascular) or tumours, as well as the study of tumour progression or metastasis, and methods for quantifying these different types of vesicles with the highest possible accuracy are needed. Another field of great interest related to these vesicles involves the development of personalised therapies based on the extraction of autogenous vesicles, modification of their cargo with active ingredients or nucleic acids, and their re-implantation in the patient after introduction of the cargo. There are also new cosmetic and pharmaceutical products under development, based on the use of extracellular vesicles isolated from different sources such as algae cultures, milk or cell lines.

Various methods exist for the analysis and characterisation of cellular vesicles or nanoparticles, such as transmission electron microscopy (TEM), dynamic light scattering (DLS) or asymmetric fractional flow analysis. In the case of quantification, the methods used require expensive and complex equipment such as Enzyme Linked Immunosorbent Assays (ELISA) to do quantitative determination of biomarkers in the extracellular vesicles, tuneable resistive pulse sensing or nanoparticle tracking analysis (NTA). Tuneable resistive pulse sensing is currently one of the most sensitive and accurate techniques whose principle consists of two electrodes containing cellular fluids between which is a non-conductive polyurethane membrane, which contains a single cone-shaped pore of around one micron in size. The samples to be analysed are diluted in a buffer with a conductive electrolyte and dispersed around the cell. When an electrical potential is applied, these particles pass individually through the pore generating a resistance pulse, which is measured and can be related to the volume of the particle that caused it. However, this requires expensive equipment, as well as specialised personnel and a central laboratory.

There is therefore a need for an affordable, portable and easy to use system and method for nanoparticle quantification or screening that can compare in detection speed and precision to the current ones, so that the nanoparticle quantification can be spread to rutine analysis in research or control of environmental samples and everyday products such as food products or pharmaceutical and cosmetic preparations, in order to protect the consumer.

### SUMMARY OF INVENTION

A first aspect of the invention refers to a system for determining the presence and/or concentration of nanoparticles in a sample comprising a device and a detection reagent. The detection reagent comprises an electrochemical probe, and the device comprises:
a) an electrochemical sensor characterized by comprising at least one working electrode and a second electrode;
b) a sample application area in fluid communication with the electrochemical sensor;
c) a membrane immobilized on said working electrode, the membrane being characterized by comprising nanochannels that control the sample (20) access to the working electrode; and
d) means for determining the difference in the electrochemical state between the working electrode and the second electrode.

In the system, the presence and/or concentration is determined from the change in the difference in the electrochemical state caused by the blockage of the nanochannels by the presence of the nanoparticles in the sample.

According to a preferred embodiment, the second electrode is a counter electrode, wherein the difference in the electrochemical state between the working electrode and the counter electrode is the faradaic current between the working electrode and the counter electrode produced by a redox reaction of the electrochemical probe.

According to another preferred embodiment, the diameter of the nanochannels of the membrane are comprised between 50 and 1000 nm, preferably between 50 and 100 nm.

According to another preferred embodiment, the diameter of the nanochannels of the membrane are at least of the size of the nanoparticles whose presence and/or concentration is being determined, preferably at most 25% greater.

According to a preferred embodiment wherein the second electrode is a counter electrode, the counter electrode is a silver/silver chloride electrode and the means is a potentiostat configured to provide a potential comprised between -0.5 V and 1V.

According to another preferred embodiment wherein the second electrode is a counter electrode, the means is a potentiostat configured to perform a cyclic or linear voltammetry, preferably wherein the voltage is increased and/or decreased between two values at a rate comprised between 25 mV/s and 100 mV/s. In a more preferred embodiment, the potentiostat is configured to perform a differential pulse voltammetry or square wave voltammetry

According to another preferred embodiment, the electrochemical probe comprises any of the materials selected from the following list: potassium ferrocyanide, hexamine ruthenium (III) chloride, and ferrocene soluble derivatives such as ferrocene methanol.

According to another preferred embodiment, the electrochemical sensor is a screen-printed carbon electrode (SPCE).

According to another preferred embodiment, the working electrode and/or the second electrode comprise any of the materials selected from the following list: carbon, platinum, gold and bismuth.

A second aspect of the invention refers to the use of a system according to any one of the systems of the first aspect of the invention to determine the presence and/or concentration of nanoparticles and/or nanovesicles in a food sample.

An alternative embodiment of the second aspect of the invention refers to the use of a system according to any one of the systems of the first aspect of the invention, to determine the presence and/or concentration of nanoparticles and/or nanovesicles in an environmental or cosmetic or pharmaceutical sample.

A third aspect of the invention refers to a method for determining the presence and/or concentration of nanoparticles in a sample. The method comprises the steps of:
a) providing a detection reagent according to any reagent of the first aspect of the invention onto the sample application area of a device according to any one of the devices of the first aspect of the invention;
b) providing the sample onto the sample application area of the device;
c) determining the difference in the electrochemical state between the working electrode and the second electrode; and
d) determining the presence and/or concentration of the nanoparticles in the sample based on the difference in the electrochemical state determined in step c).

In an alternative embodiment, the method comprises the steps of:
a) providing a detection reagent according to any reagent of the first aspect of the invention onto the sample application area of a device according to any one of the devices of the first aspect of the invention;
b) determining the difference in the electrochemical state between the working electrode and the second electrode;
c) providing the sample onto the sample application area of the device;
d) determining the difference in the electrochemical state between the working electrode and the second electrode; and
e) determining the presence and/or concentration of the nanoparticles in the sample based on the comparison of the difference in the electrochemical state determined in step b) and in step d).

According to a preferred embodiment of any of the embodiments of the third aspect of the invention, the second electrode is a counter electrode and wherein the difference in the electrochemical state is the faradaic current between the working electrode and the counter electrode.

### BRIEF DESCRIPTION OF DRAWINGS

**Fig. 1****:** shows a diagram of a system for determining the presence and/or concentration of nanoparticles in a sample according to one or more embodiments of the invention.
**Fig. 2****:** shows a diagram of a method for determining the presence and/or concentration of nanoparticles in a sample according to one or more embodiments of the invention.
**Fig. 3****:** shows a scheme of preparation of the membrane over the electrochemical sensor according to one or more embodiments of the invention.
**Fig. 4****:** shows a voltammetry graph of a system before and after washing it according to one or more embodiments of the invention.
**Fig. 5****:** shows a voltammetry graph of a system for different nanoparticle concentrations according to one or more embodiments of the invention
**Fig. 6****:** shows a table for the voltammetric results of a system for different nanoparticle concentrations according to one or more embodiments of the invention.
**Fig. 7****:** shows the calibration curve of a system according to one or more embodiments of the invention.
**Fig. 8****:** shows another table for the voltammetric results of a system for different nanoparticle concentrations according to one or more embodiments of the invention.
**Fig. 9****:** shows the calibration curve of another system according to one or more embodiments of the invention.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of' excludes any element, step, or ingredient not specified in the claim element. When used herein, "consisting essentially of does not exclude materials or steps that do not materially affect the basic and novel characteristics of the claim.

The term "nanoparticle" refers to any particle in the nanometric scale, therefore ranging between 1 and 1000nm. They may be naturally synthesized such as extracellular vesicles, or artificially synthesized such as Au, Ag, C, Cu, liposomes, micelles, latex, iron oxide and other metal oxide nanoparticles or quantum dots and microplastics.

The term "reagent" refers to a substance or compound configured to cause a chemical reaction or to test if one occurs.

The term "electrochemical probe" refers to a compound with capacity for electron exchange with an electrode, configured to test the electrochemical properties of the sensor.

The term "difference in the electrochemical state" refers to any difference between the state of two electrodes or their environment, such as presence of a differential, current flowing from one electrode to the other one or any other chemical difference that can be electrically measured.

The term "electrochemical sensor" refers to any sensor or cell which employs chemical interactions with charges and electrons in an electrode interface to monitor the presence and/or concentration of a substrate of said changes by measuring the difference in the electrochemical state between such electrode and another counter or reference electrode. These interactions might be Faradaic and/or non-Faradaic. When there are Faradaic processes involved, the counter electrode performs a reaction to balance the electrical charges. In potential-based measurements, when there are not Faradaic processes involved, the counter electrode might behave as a reference (or pseudo-reference) electrode. Also, the term "electrochemical cell" (i.e., the ensemble of working and counter electrode and the polyelectrolyte layer) might be used indistinctively unless stated otherwise.

The term "working electrode" is preferably understood as the electrode where the main interaction (transfer of electrons) with the target analyte or electrochemical probe will occur.

The term "counter electrode" is preferably understood as the electrode providing a complementary faradaic reaction to the reaction carried out at the working electrode.

The term "reference electrode" is preferably understood as the electrode that serves as reference to establish a stable level of potential to which the changes in the working electrode potential are referred to.

### DESCRIPTION OF THE EMBODIMENTS

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

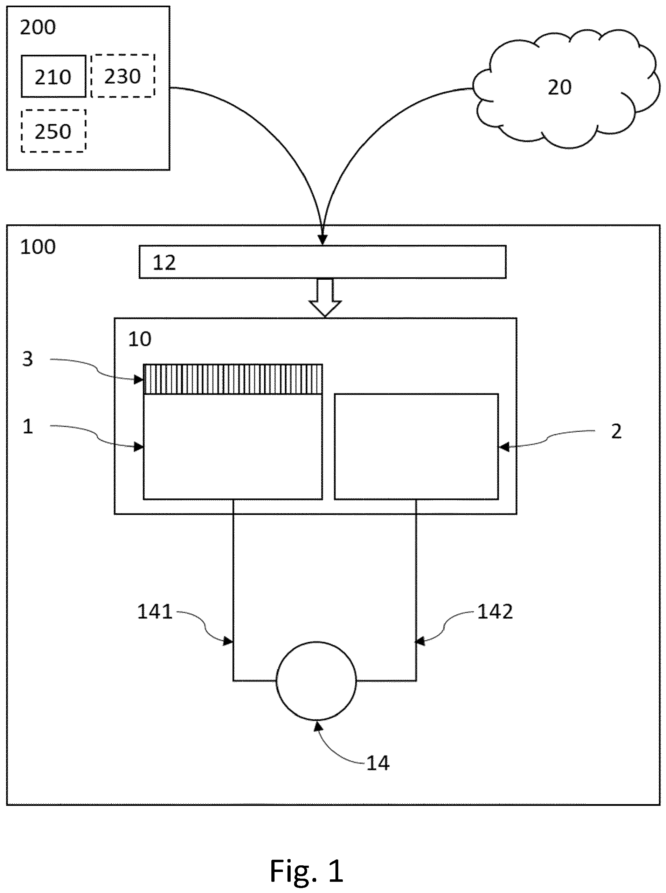

A first aspect of the invention refers to a system for determining the presence and/or concentration of nanoparticles in a sample (20). As shown in Fig. 1, the system comprises a device (100) and a detection reagent (200).

The detection reagent (200) comprises an electrochemical probe (210). The electrochemical probe is a compound with capacity for electron exchange with an electrode and preferably reversible behavior, which are known to be suitable for determining the reactivity of an electrode. There are many electrochemical probes such as potassium ferrocyanide, hexamine ruthenium (III) chloride, and ferrocene soluble derivatives such as ferrocene methanol.

The device (100) comprises an electrochemical sensor (10), a sample application area (12), a membrane (3) and means (14) for determining the difference in the electrochemical state of the electrochemical sensor (10).

The electrochemical sensor (10) is characterized by comprising at least one working electrode (1) and a second electrode (2). The electrochemical sensor (10) may be a current-based sensor, such as an amperometric or voltammetric sensor, in which the second electrode (2) is the counter electrode and a further reference or pseudo-reference electrode is provided; as well as a potentiometric sensor, in which the second electrode (2) is the reference electrode. Therefore, it is noted that the second electrode (2) may be any electrode from the electrochemical sensor (10) other than the working electrode (1), and that in some embodiments, the electrochemical sensor (10) may require other electrodes to work.

Although in Fig. 1 the working electrode (1) and the second electrode (2) are placed laterally to each other and they resemble the same rectangular regular shape, it is noted that in some embodiments, the shape and size of the front and back electrodes may differ in several ways. For example, they maybe radially disposed, with one circular electrode, such as the working electrode (1) comprised within a ring-shaped electrode, such as the second electrode (2). The skilled person would therefore appreciate several suitable alternatives for the position and shape of the working electrode (1) and the second electrode (2).

The sample application area (12) is in fluid communication with the electrochemical sensor (10). The sample application area (12) may be any area of the device (100) in fluid communication with the electrochemical sensor (10). Thus, in some embodiments, the sample application area (12) is an area located above just before or above the electrochemical sensor (10), while in other embodiments the sample application area (12) may be relatively apart from the electrochemical sensor (10) but still fluidly communicated with it. For such purpose the device may further comprise a microfluidic channel or an adsorbent material configured to fluidly communicate the sample (20) provided to the electrochemical sensor (10) by surface tension or osmosis. This may be for example the case wherein the device (100) is implemented in a lateral flow assay - like form, wherein the electrochemical sensor (10) would never be accessible to the user. In some other embodiments, the sample application area (12) is the very same electrochemical sensor (10). Thus, it is noted that according to the present invention, the sample application area (12) may be any area of the device (100) as long as it is in fluid communication with the electrochemical sensor (10) such that samples can be provided from the sample application area (12) to the electrochemical sensor (10). Throughout the present disclosure, it is understood that with the provision of the detection reagent and/or sample to the sample application area (12) it is provided at least to the working electrode (1) and the second electrode (2) of the electrochemical sensor (10), effectively fluidly communicating them.

Once the detection reagent (200) is provided to the sample application area (12), the detection reagent (200) serves as the medium where the electrons of the electrodes (1,2) and the electrochemical probe (210) are exchanged at the electrochemical sensor (10).

The membrane (3) is immobilized on said working electrode (1) and characterized by comprising nanochannels that control the sample access to the working electrode (1). The membrane (3) is non-conductive and may be immobilised on the working electrode (1) by different means. For example, the membrane (3) may comprise an adhesive configured to stick the membrane (3) to the working electrode (1) or preferably to the surroundings of the working electrode (1). Alternatively, the membrane (3) and the working electrode (1) may be physically attached together through a casing comprising both and fixing both in contact.

The nanochannels of the membrane (3) control the sample access to the working electrode (1) such that the only way to access the working electrode (1) is through the membrane (3), particularly through its nanochannels. The nanochannels are essentially cylindrical, with one end open for the sample to enter and the other end closed by the working electrode (1) surface. Nevertheless, it is noted that while the nanochannel direction is preferably perpendicular to the working electrode (1) surface, in some embodiments, the nanochannels may be slightly or significantly obliquely defined with respect to the surface of the working electrode (1) closing one of the ends. It may be that in some embodiments, all the surfaces of the working electrode (1) not covered by the membrane (3) are isolated from the detection reagent (200) and sample, for example with a tape or an isolating piece. In other embodiments, the working electrode (1) is covered with the membrane (3) in more than one face.

Different embodiments may comprise different types of nanochannels arranged in different fashions. In some embodiments, the membrane (3) may comprise two or more nanochannels, and the nanochannels may be all equal or different. The nanochannels may have different diameters and heights. Thus, in some embodiments, the membrane may comprise nanochannels of different diameter and/or height, such that different nanoparticles can be sensed. In some other embodiments, the nanochannels may comprise variable diameters along each nanopore, therefore making such nanochannels compatible to sense more than one specific diameter.

As above mentioned, the means (14) determine the difference in the electrochemical state between the working electrode (1) and the second electrode (2). The means (14) is connected to the working electrode (1) and the second electrode (2) through a couple of low resistance wires (141, 142) that connect it with each electrode (1,2) so that it can determine the difference in their electrochemical state. It must be noted that several means (14) can achieve such effect, such as an ammeter or a voltmeter, as long as the difference in the electrochemical state between the working electrode and the second electrode can be determined. It must be also noted that additional electrical or electronic means can be added in the electric circuit to facilitate the measurements in any of the embodiments described herein.

The electrochemical state may comprise either the current flowing through both electrodes (1,2) or the voltage between both electrodes (1,2) or between both electrodes (1,2) and a third reference electrode caused by electrochemical reactions produced by the presence of nanoparticles in the sample. This comprises faradaic (redox) reactions.

The presence and/or concentration in the sample is determined from the change in the determined difference in the electrochemical state, caused by the blockage of the nanochannels by the nanoparticles in the sample (20). Either when there is a current through both electrodes (1,2) or a voltage is present between both electrodes (1,2), independently of the origin of this current or voltage, the electrochemical probe (210) comprised within the detection reagent (200) will diffuse through the membrane nanochannels and interact with the working electrode (1), generating a difference in the electrochemical state between both electrodes, either a current flowing from one to the other one or a voltage between both. This is because the electrochemical probe (210) is capable of exchange electrons with the working electrode (1), usually through a redox reaction. It is noted that when the electrochemical sensor only comprises a working electrode (1) and a counter electrode (2) and they are both comprised by the same materials, the means (14) therefore preferably provides the necessary electrical current and/or voltage between such working electrode (1) and counter electrode (2) to ensure the electrons of the electrodes (1,2) and the electrochemical probe (210) are exchanged at the electrochemical sensor (10).

When a sample comprising nanoparticles is added, they also diffuse into the membrane nanochannels and block the electrochemical probe (210) interaction with the working electrode (1). Therefore, a decrease in the electrochemical state between both electrodes is observed, which is proportional to the concentration of nanoparticles or nanovesicles. Thus, a change in the difference in the electrochemical state between both electrodes (1,2) is indicative of the presence and/or concentration of nanoparticles in the sample provided.

While the presence of nanoparticles in the sample can be derived from any change in the difference in the electrochemical state between both electrodes (1,2), the concentration may be further derived from a correlation between the difference in the electrochemical state and the concentration of such analyte, for example through a lookup table or a mathematical formula.

Advantageously, this system comprising the device (100) and the detection reagent (200) due to its simplicity in materials is an affordable, portable, and simple to use system for nanoparticle quantification. It is portable as it requires no complex analysis technologies and it's easy to use, as it only requires the provision of the detection reagent (200) and the sample (20) on the device. This enables screening at point-of-use or in settings with low resources whereof other more complex and expensive technologies are not available, therefore, providing an affordable way of determining the presence and/or concentration of nanoparticles in a sample.

In a preferred embodiment of the first aspect of the invention, the second electrode (2) is a counter electrode. Thus, this preferred embodiment, the difference in the electrochemical state between the working electrode (1) and the counter electrode (2) is the faradaic current between the working electrode (1) and the counter electrode (2) produced by a redox reaction of the electrochemical probe (210).

It is noted that in some embodiments of this preferred embodiment, the potentiostat provides a voltage between the working electrode (1) and a reference electrode, while in other embodiments, the potentiostat does not provide any voltage between the working electrode (1) and a reference electrode, and therefore the system is self-powered in the sense that the potentiostat does not require to provide any voltage in order for the faradaic current between the working electrode (1) and the counter electrode (2) to occur. It is noted that in the later embodiments, the reference electrode may be therefore not present.

In this preferred embodiment, the presence and/or concentration in the sample is determined from the change in the determined difference in the electrochemical faradaic current, caused by the blockage of the nanochannels by the nanoparticles in the sample (20). When there is a voltage applied to the working electrode (1) with respect to a current reference electrode, the electrochemical probe (210) comprised within the detection reagent (200) will diffuse through the membrane nanochannels and interact with the working electrode (1) through a redox reaction, generating a current flowing between the working electrode (1) and the counter electrode (2) as a result of the difference in the electrochemical state between both electrodes, as the electrochemical probe (210) exchanges electrons with the working electrode (1).

When a sample comprising nanoparticles is added, they also diffuse into the membrane nanochannels and block the electrochemical probe (210) redox reaction with the working electrode (1). Therefore, a decrease in the faradaic current between both electrodes is observed, which is proportional to the concentration of nanoparticles or nanovesicles. Thus, a change in the faradaic current between both electrodes (1,2) is indicative of the presence and/or concentration of nanoparticles in the sample provided.

While the presence of nanoparticles in the sample can be derived from any decrease in the faradaic current between both electrodes (1,2), the concentration may be further derived from a correlation between the Faradaic current and the concentration of such analyte, for example through a lookup table or a mathematical formula.

In some embodiments of this preferred embodiment, the electrochemical probe (210) is oxidised at the working electrode (1), while in other embodiments, the electrochemical probe (210) is reduced at the working electrode (1). For example, the electrochemical probe (210) may be only oxidised when an anodic potential is provided, while when a cationic potential is provided it will be only reduced at the working electrode (1). It can also happen that for some electrochemical probe (210), such as in the case of potassium ferrocyanide the reaction is reversible depending on the voltage applied to the working electrode (1). For instance, the electrochemical probe (210) may have a switching potential, the switching potential defining a potential threshold wherein the electrochemical probe (210) changes its reaction with the working electrode (1) from oxidation to reduction and vice versa. This switching potential depends on the electrochemical properties of the electrochemical probe (210).

According to another preferred embodiment of the first aspect of the invention, the diameter of the nanochannels of the membrane (3) are comprised between 50 and 1000 nm.

It is noted that the diameter of the nanochannels is defined as the diameter of the cylindrical equivalent of the nanochannels. Thus, in those cases wherein the nanochannels are irregular in shape, the average diameter of these nanochannels is considered to be their diameter. It is also noted, that in those cases, wherein the nanochannel comprises a variable diameter along the channel, for example with a smaller diameter on the working electrode (1) end and a bigger diameter on the free end wherein the reagent and the sample can enter the nanochannel, the diameter may be any diameter along the nanochannel. This is, according to the present embodiment, as long as the nanochannel comprises an equivalent diameter comprised between 50 and 1000nm anywhere along its length (i.e., between the free end and the working electrode (1) end) it can be considered that the nanochannel comprises a diameter between 50 and 1000nm.

Advantageously, this allows for the system to detect the presence and/or concentration of nanoparticles in the samples. When these samples are representative of the environment of the subject, such as soil, water or air, it helps determining and quantifying the presence of these elements in the environment, effectively enabling taking preventive measures against the toxic effects of these nanoparticles for animals and humans. Additionally, this allows for the quantification of extracellular vesicles, which can serve as biomarkers for diverse respiratory and cardiovascular diseases as well as for tumours. For example, it enables tracking tumour metastasis.

In a more preferred embodiment, the diameter of the nanochannels of the membrane (3) are at most 100 nm. Advantageously, this allows the monitoring and quantification of materials smaller than 100 nm in food or cosmetic or pharmacological compounds. These are known control tests aimed at protecting consumers, for example to detect nanoparticles that macrophages at the liver may not be able to process (i.e., smaller than 100 nm), and they therefore may accumulate in the liver..

According to another preferred embodiment of the first aspect of the invention, the diameter of the nanochannels of the membrane (3) are at least of the size of the nanoparticles whose presence and/or concentration is being determined. As the mechanism of action requires for the nanoparticles in the sample to diffuse into the membrane nanochannels and block the electrochemical probe (210) interaction with the working electrode (1), it is preferred for the nanochannels at least of the size of the nanoparticles whose presence and/or concentration is being determined.

In some embodiments of this preferred embodiment, it may be that the membrane (3) comprises nanochannels with different diameters, such that the system can determine the presence and/or concentration of several nanoparticles of different diameter. In this embodiment, as long as one of the nanochannels has a diameter of at least of the size of the nanoparticles it would be able to determine the presence and/or concentration of such nanoparticle.

Advantageously, this allows for the device (100) to be able to determine the diameter of the particles whose presence and/or concentration is being determined. For example, if a device (100) has a membrane (3) with nanopores with 200 nm, we know it will change the electrochemical state between the electrodes (1,2) if a nanoparticle of at most 200 nm is present.

In a more preferred embodiment, the diameter of the nanochannels of the membrane (3) are at most 25% greater than the diameter of the particles to determine the presence and/or concentration, more preferably, at most 15% greater, even more preferably at most 10% greater. If the diameter of the nanochannels of the membrane (3) are too big compared to that of the nanoparticles, the nanoparticles may not be able to effectively block such nanochannels, and the electrochemical probe (210) interaction with the working electrode (1). This would therefore hinder the determination of the presence and/or concentration of particles from a certain diameter of interest.

Advantageously, this allows for the device (100) to be able to determine the particles in a determined size, without having the reading interfered by bigger particles to those of interest. Thus, it allows a fine tuning of the membrane to the size of particles of interest. When more than one of these systems are used, each with different nanochannel diameters, the size of the nanoparticles in the sample can be characterised also according to their diameter.

According to another preferred embodiment of the first aspect of the invention, the detection reagent (20) further comprises Tris buffer (230) and/or sodic sulphate (250) as a medium. Advantageously, this provides an electrolytic medium to the detection reagent (20) that is particularly favourable for its wetting properties with the proposed membranes nanochannels, therefore improving the signal quality.

According to another preferred embodiment of the first aspect of the invention the reference electrode is a silver/silver chloride electrode, and the means (14) is a potentiostat (14) configured to provide a potential comprised between -0.5 V and 1V.

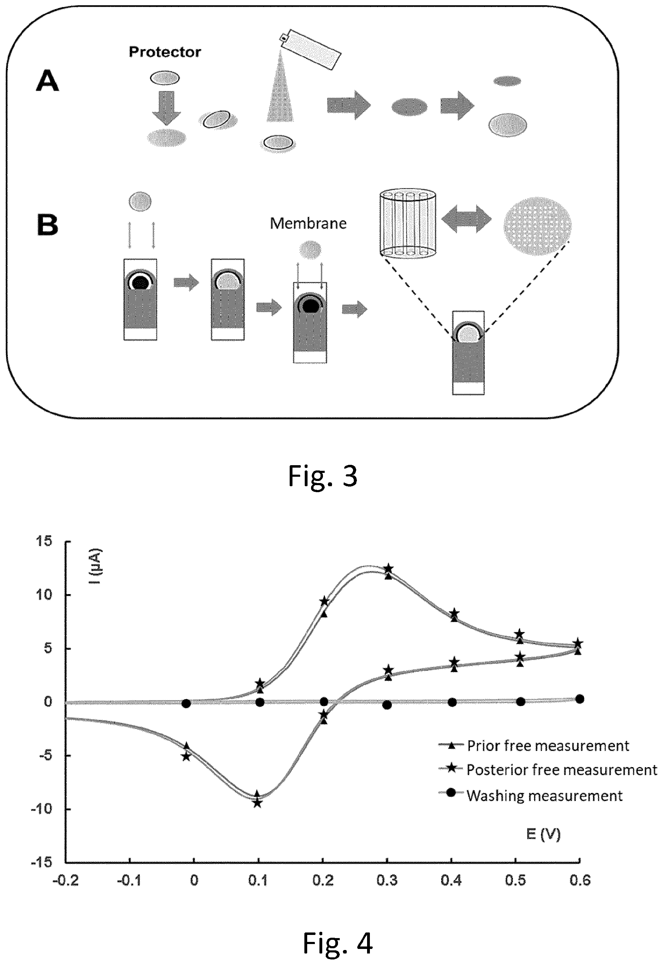

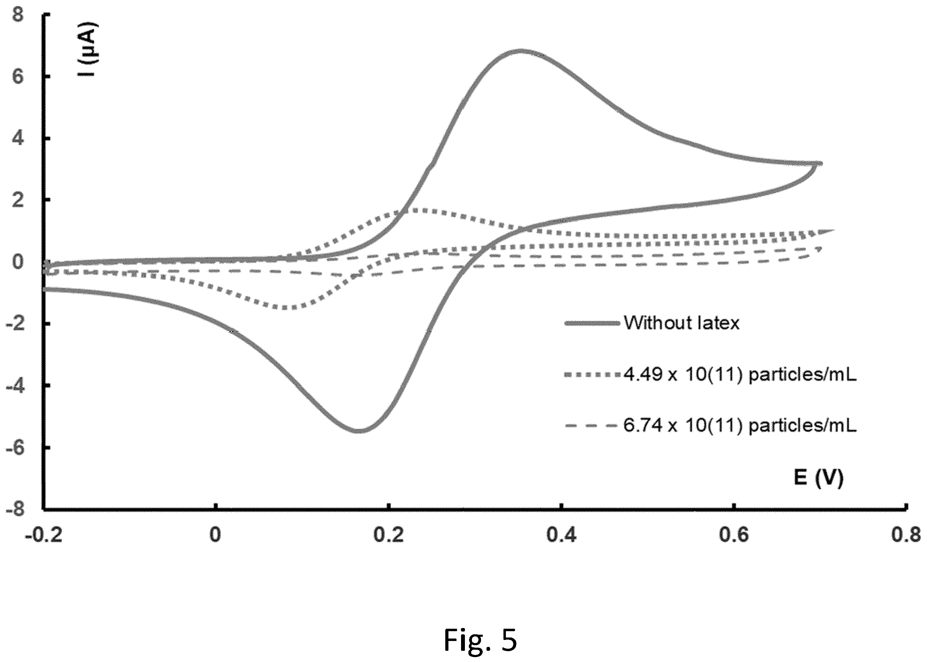

Due to the electrochemical dependence of this system, is to be expected that the material of the reference electrode affects the reaction of the working electrode (1) at a determined voltage. Thus, each reference electrode material has its own voltage range wherein the electrochemical probe (210) reacts with the working electrode either by reduction or oxidation. For silver/silver chloride reference electrodes is has been shown that a range between -0.5 and 1V is enough to cover the whole spectrum of the electrochemical probe (210) redox reactivity, as shown in Example 1 with respect to Figs. 4 and 5. Advantageously, this allows for the system to work at any of the voltages wherein the electrochemical probe (210) reacts with a working electrode (1) with a silver/silver chloride reference electrode.

In a particular preferred embodiment, the potentiostat (14) is configured to provide a potential comprised between -0.2V and 0.7V. Advantageously, this voltage range makes the system work in the most sensitive range of silver/silver chloride reference electrodes, where the redox reaction by the electrochemical probe (210) is the greatest.

It is however noted, that for other reference electrode materials, such as carbon, or gold, the voltage ranges of the potentiostat (14) may be different. However, given the above disclosure with respect to silver/silver chloride electrodes, it is to be expected that the range for these other electrode materials to be easily derived.

According to another preferred embodiment of the first aspect of the invention, the means (14) is a potentiostat (14) configured to perform a cyclic and or linear voltammetry. Cyclic voltammetry consists on continuously increasing and decreasing the applied voltage between the working electrode (1) and a reference electrode, while the current between the working electrode (1) and the counter electrode (2) is registered. Linear voltammetry consists on a single continuous increase or decrease of the applied voltage between the working electrode (1) and a reference electrode between two values. Preferably, when the reference electrode is a silver/silver chloride electrode, the applied voltage between the working electrode (1) and a reference electrode is comprised between -0.5 and 1 V, more preferably between -0.2 and 0.7V.

Advantageously, performing such a cyclic or linear voltammetry allows to characterise the reactivity of a particular electrode material and electrochemical probe (210) in a reagent (200), which in turn allows to determine the optimum voltages that provide the biggest sensitivity to the system in terms of current.

In a more preferred embodiment, the voltage is increased and/or decreased between two values at a fixed rate comprised between 25mV/s and 100 mV/s, more preferably between 25mV/s and 75 mV/s, even more preferably, between 40mV/s and 60 mV/s .

In a further preferred embodiment of the first aspect of the invention, the potentiostat (14) is configured to perform a differential pulse voltammetry or square wave voltammetry.

The differential pulse voltammetry consists on providing voltage pulses at a certain voltage, preferably at voltages wherein the redox reaction by the electrochemical probe (210) is highest, i.e., when the sensitivity is greatest, to determine the presence and/or concentration of nanoparticles at a given time. Similarly, square wave voltammetry consists on a square wave alternating between two voltages, such that the differential between both voltage values provides the greatest sensitivity to determine the presence and/or concentration of nanoparticles.

For example, the potentiostat (14) may provide a differential pulse voltammetry with a potential step of 4 mV, a potential pulse of 25 mV, a pulse time: 0.2 s and a sweep rate of 0.05 V/s. Alternatively, the potentiostat (14) may provide a square wave voltammetry with a potential step of 4 mV, a potential amplitude of 0.02V, and a frequency of 2Hz. Therefore, the skilled person may appreciate there are different ways in which differential pulse voltammetry or square pulse voltammetry may be provided.

Advantageously, the potentiostat (14) being configured to perform a differential pulse voltammetry or square wave voltammetry allows for a faster and alternative way of quantifying the presence and/or concentration of nanoparticles along time.

In another preferred embodiment of the first aspect of the invention, the wherein the electrochemical probe (210) comprises any of the materials selected from the following list: potassium ferrocyanide, hexamine ruthenium (III) chloride, and ferrocene soluble derivatives such as ferrocene methanol. Advantageously, these are electrochemical probes with high capacity for electron exchange with the working electrode (1) and that are reversible, i.e., can be either reduced or oxidized depending on the voltage applied.

In another preferred embodiment of the first aspect of the invention the electrochemical sensor (10) uses a screen-printed carbon electrode (SPCE) as electrochemical cell. Advantageously, this further makes the system, particularly the device (100) to be low cost and to be easily modified upon requirement, while maintaining a great reproducibility. Moreover, additional nanomaterials such as carbon nanotubes, graphene, and or metallic nanoparticles can be directly integrated to the sensor to facilitate the measurements in any of the embodiments described herein. In addition, the electrochemical sensor (10) may further comprise the electrical means (141, 142) for directly connecting the means (14) as part of the SPCE, which further facilitates the measurements in any of the embodiments described herein.

In another preferred embodiment of the first aspect of the invention the working electrode (1) and/or the counter second electrode (2) comprise any of the materials selected from the following list: carbon, platinum, gold, and bismuth.

A second aspect of the invention refers to the use of the system for determining the presence and/or concentration of nanoparticles in a sample (20) according to any one of the embodiments of the first aspect of the invention to determine the presence and/or concentration of nanoparticles and/or nanovesicles in a food sample (20).

Advantageously, this allows for the system to detect the presence and/or concentration of nanoparticles in food samples such as microplastics and determining and quantifying the presence of these elements in the environment, effectively enabling taking preventive measures against the toxic effects of these nanoparticles for animals and humans. Otherwise, these nanoparticles can cross the membrane of the cells in the body, or accumulate in tissues, which is a problem for metals such as silver or lead.

An alternative embodiment of the second aspect of the invention refers to the use of the system for determining the presence and/or concentration of nanoparticles in a sample (20) according to any one of the embodiments of the first aspect of the invention to determine the presence and/or concentration of nanoparticles and/or nanovesicles in an environmental or cosmetic or pharmaceutical sample (20).

Advantageously, this allows for the system to detect the presence and/or concentration of nanovesicles in cosmetic or pharmaceutical samples. The naturally produced extracellular vesicles have a role as mediators between cells for the transfer of substances necessary for the maintenance of functions in organisms. In this use the system of the first aspect of the invention can be useful to control the nanoparticle concentration at the development of personalised therapies based on the extraction of autogenous extracellular vesicles, modification of their cargo with active ingredients or nucleic acids, and their re-implantation in the patient after introduction of the cargo. The nanovesicles having a size around 100nm allow for the identification of the vesicles able to efficiently interact with the cell membrane, therefore being especially suitable for delivering their cargo (therapeutic principle) at the right place. Alternatively, it can be used to monitor biomarkers in various types of diseases (immunological, respiratory, cardiovascular) or tumours, as well as the study of tumour progression or metastasis, and methods of quantifying these different types of vesicles with the highest possible accuracy are needed.

This device could be useful at the current research on biological extracellular vesicles field.

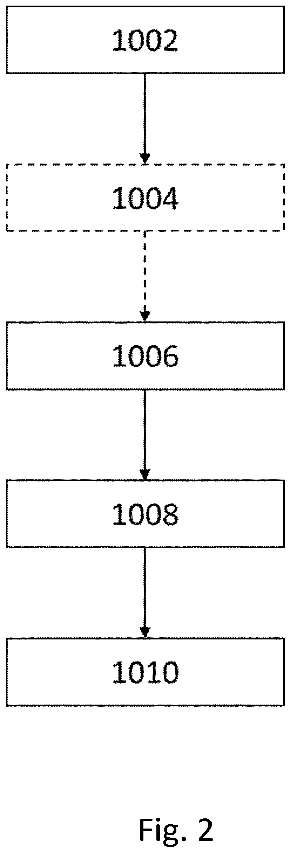

A third aspect of the invention refers to a method for determining the presence and/or concentration of nanoparticles in a sample (20). As shown with respect to Fig. 2 the method comprises the following steps:
a) providing a detection reagent (200) according to any one of the detection reagents (200) of the first aspect of the invention onto the sample application area (12) of a device (100) according to any one of the devices (100) of the first aspect of the invention;
b) determining the difference in the electrochemical state between the working electrode (1) and the second electrode (2);
c) providing the sample (20) onto the sample application area (12) of the device (100);
d) determining the difference in the electrochemical state between the working electrode (1) and the second electrode (2); and
e) determining the presence and/or concentration of the nanoparticles in the sample (20) based on the comparison of the difference in the electrochemical state determined in step b) and in step d).

As shown in Fig 2, in step 1002, a detection reagent (200) is provided to the sample application area (12) of a device (100). As the sample area (12) is fluidly communicated with the electrochemical sensor (10), this in turn means that the detection reagent (200) is provided to the electrochemical sensor (10). As the detection reagent (200) comprises the electrochemical probe (210), the electrochemical probe (210) would, upon certain electrochemical conditions, create a difference in the electrochemical state between the working electrode (1) and the second electrode (2). These electrochemical conditions may be achieved by the provision of a current or a voltage to the electrochemical sensor, either through an external source or self-powered, based on the energy generated by a biochemical reaction in the detection reagent (200).

Then, in step 1004, using the means (14) this difference in the electrochemical state between the working electrode (1) and the second electrode (2) is determined.

Afterwards, in step 1006, the sample (20) is also provided onto the sample application area (12) of the device (100). As with the detection reagent (200), as the sample area (12) is fluidly communicated with the electrochemical sensor (10), the sample is therefore provided to the electrochemical sensor (10). As previously mentioned, if nanoparticles are present in the sample (20), the nanoparticles will diffuse into the membrane nanochannels and block the electrochemical probe (210) interaction with the working electrode (1), producing a change in the difference in the electrochemical state between the working electrode (1) and the second electrode (2).

Then, in step 10008, using the means (14) the difference in the electrochemical state between the working electrode (1) and the second electrode (2) is determined.

Lastly, in step 1010, the presence and/or concentration of the nanoparticles in the sample (20) is determined based on the comparison of the differences in the electrochemical states of the electrodes (1,2) determined in steps 1004 and 1008. The presence of nanoparticles may just be computed as discrete value, wherein if the comparison shown no change in the difference in the electrochemical states, then no nanoparticles are found in the sample, otherwise, it can be determined that there are nanoparticles in the sample.

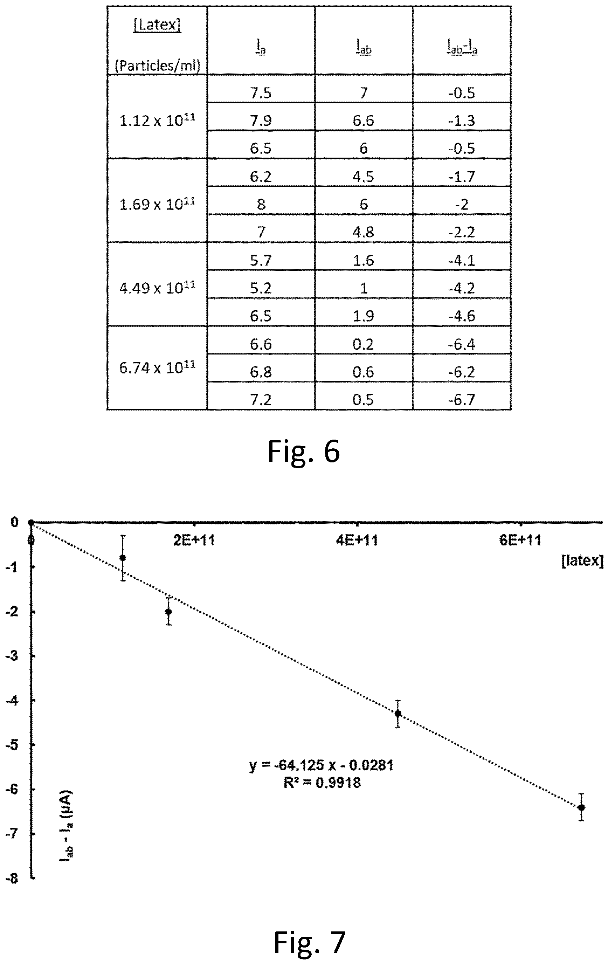

The concentration of the nanoparticles can be computed based on such comparison when a change is shown. This may be done for example by using a lookup table, that correlates the change in the difference in the electrochemical state values, to a particular concentration of nanoparticles in the sample. An example of a correlation graph from such a lookup table can be seen in Fig. 7 as explained in Example 1. Alternatively, a mathematical formula may be used. This may be computed from a previous test in which at least three samples are obtained in which one represents a sample without nanoparticles.

An alternative embodiment of the third aspect of the invention refers to a method for determining the presence and/or concentration of nanoparticles in a sample (20). As shown with respect to Fig. 2 the method comprises the following steps:
a) providing a detection reagent (200) according to any one of the detection reagents (200) of the first aspect of the invention onto the sample application area (12) of a device (100) according to any one of the devices (100) of the first aspect of the invention;
b) providing the sample (20) onto the sample application area (12) of the device (100);
c) determining the difference in the electrochemical state between the working electrode (1) and the second electrode (2); and
d) determining the presence and/or concentration of the nanoparticles in the sample (20) based on the difference in the electrochemical state determined in step c).

It is noted, that in this case, it is not needed to firstly determine an electrochemical state for just the detection reagent (step 1004), and later on a second electrochemical state when the sample is added (step 1008). This is because, in some embodiments, the method may be able to determine the presence and/or concentration just from the determined difference in the electrochemical state between the working electrode (1) and the second electrode (2) when a lookup table and/or a relationship curve between the measured current and the concentration of particles is preestablished. Moreover, it is noted that the initial current registered buy the means (14) it's the greatest as it corresponds to a clean or blank medium (as the nanoparticles have not yet blocked the nanochannels). Therefore, in these embodiments, step 1004 is optional.

To do so, a lookup table or mathematical formula correlating the difference in the electrochemical state values, such as voltage or current, to a particular concentration of nanoparticles in the sample. This may be computed from a previous test in which at least three samples are obtained in which one represents a sample without nanoparticles. As in the previous embodiment, the presence of nanoparticles may just be computed as discrete value, wherein if the value of the difference in the electrochemical state is equal to the known value when no nanoparticles are in the sample, then no nanoparticles are found in the sample, otherwise, it can be determined that there are nanoparticles in the sample

In a preferred embodiment of any of the embodiments of the third aspect of the invention, the method is applied to a device (100) wherein the second electrode (2) is a counter electrode (2) and wherein the difference in the electrochemical state is the faradaic current between the working electrode (1) and the counter electrode (2).

Thus, the lookup table may represent the current intensity or current intensity change representing a certain concentration of nanoparticles in the sample provided in step 1006.

The invention is described below by the following examples, which must be considered as merely illustrative and in no case limiting of the scope of the present invention.

### EXAMPLES

### EXAMPLE 1: Detection of 300nm latex beads using potassium ferrocyanide

### Materials

### Electrodes

The screen-printed carbon electrodes (SPCE) were purchased from Metrohm Dropsens S.L. (DRP-110, Spain). They consist of a combination of a working and an auxiliary electrode made of carbon with a silver pseudo reference electrode. A DSC connector (DRP-DSC) is used as the method of connection between the screen-printed electrode and the potentiostat.

### Reagents

- Potassium ferrocyanide trihydrate was purchased from Merck KGaA (Germany). Tris(hydroxymethyl)aminomethane and sodium sulphate were purchased from Sigma-Aldrich (India).
- Ultrapure water (milliQ) was purchased from a milli-q purification system.
- A 0.1 M Tris-nitric solution pH= 7 was prepared with 1 M Na2SO4 as inert electrolyte. Electrochemical measurements on the membrane electrodes were performed with a 1 mM potassium ferrocyanide solution in tris-nitric buffer pH=7.

### Membranes

Isophoric polycarbonate membranes (MF-Millipore Membrane Filters were purchased from Merck Life Science S.L.U. They were purchased in the following nanochannel diameter sizes 200,400 and 800 nm. A suspension of latex beads (latex beads, polystyrene) of 300 nm was purchased from Sigma-Aldrich.

### Potentiostat

Electrochemical measurements were carried out with an mStat 400 potentiostat using the software Dropview 8400. Measurements were carried out by cyclic voltammetry (E₀ = -0.2 V, E_{f} = 0.7 V, scan rate v = 50 mV). The measurements were carried out in triplicate.

### Methods

As shown in Fig. 3, firstly, the electrodes were modified by overlaying them with an adhesive-covered protective foil so that only the ceramic region is covered with adhesive (A) and being isolated from the circuit, does not interfere with the electrochemical measurements. In this way, the membrane could be positioned in such a way that it was not damaged during the adhesion process (B).

However, this process generates air bubbles that prevent the diffusion of the sample to the electrode surface. Therefore, prior to measurement, a volume of tris nitric solution pH= 7 was added to the membrane electrodes and left at 4°C until no air bubbles were observed which improved the sensitivity of the system. After this, they are ready to measure.

Consecutive measurements were made, washing and drying at room temperature until two identical measurements are obtained as shown in Fig. 4. The measurement of the blank confirmed that no ferrocyanide remained from the previous measurement that could interfere. In this way we obtained a reliable measurement of the membrane electrode, and we can corroborate that all possible changes with respect to the signal are due to the addition of any type of nanoparticle to be blocked.

A measurement of 1 mM ferrocyanide in 0.1 M tris nitric + Na₂SO₄ was performed. We calculated the oxidation intensity before adding the latex nanoparticles Iₐ, then we added 1 µL of different dilutions of the latex stock solution and let it dry. Finally, a second ferrocyanide measurement was performed to obtain a second oxidation intensity after blocking I_{ab}.

### Results

Fig. 5 shows a cyclic voltammetry between -0.2 and 0.7V for samples with no nanoparticles, and for samples with latex nanoparticles at different concentrations (4.49 × 10¹¹ particles/mL and 6.74 × 10¹¹ particles/mL). It can be seen that the presence of latex nanoparticles in the sample decreases the electrochemical difference between the working electrode and the second electrode, particularly, the Faradaic current decreases. This can be explained as the latex nanoparticles block the nanochannels so that the potassium ferrocyanide, this is, the electrochemical probe can no longer access the working electrode surface and generate such Faradaic current. Moreover, it can be seen that the greater the nanoparticle concentration in the sample, the larger the decrease in the Faradaic current produced between the working electrode and the second (counter) electrode.

Fig. 6 shows a table with the measurements before (Iₐ) and after (I_{ab}) adding a sample with nanoparticles, for different nanoparticle concentrations between 1.12 × 10¹¹ and 6.74 × 10¹¹ particles/mL . Each measurement was repeated three times. In all cases, it can be seen that for different measurements the provision of a sample with nanoparticles reduces the Faradaic current (I_{ab-}Iₐ). It can also be noted that for all three measurements, the decrease is similar, and the results are replicable.

Fig. 7 is a calibration curve obtained from the results in Fig. 6, which shows that there is a linear relationship between the reduction in Faradaic current (I_{ab-}Iₐ) and the concentration of nanoparticles in the sample, with high correlation (R = 0.9918).

Moreover, although not shown in Fig. 7, from the table in Fig. 6 it can be observed that a correlation between the Faradaic current (I_{ab}) and the concentration of nanoparticles in the sample can be also obtained which can be used to build a lookup table and/or a relationship curve between I_{ab} and the concentration of particles in the sample.

It is noted that nanoparticles in aqueous medium are surrounded by a cloud of ions that travel with them when they move in the medium, in form of a colloid. Therefore, their actual hydrodynamic radius is larger than the particle radius. This is why for latex beads 300 nm no signal was detected with membranes with 400 nm pores, while those with 800 nm were found suitable.

From the above results, it is concluded that a system according to the first aspect of the invention, through the methodology of the second aspect of the invention can determine the presence and/or concentration of nanoparticle sin a sample with high accuracy.

### EXAMPLE 2: Detection of exomes using potassium ferrocyanide

### Materials

### Electrodes

The screen-printed carbon electrodes (SPCE) were purchased from Metrohm Dropsens S.L. (DRP-110, Spain). They consist of a combination of a working and an auxiliary electrode made of carbon with a silver pseudo reference electrode. A DSC connector (DRP-DSC) is used as the method of connection between the screen-printed electrode and the potentiostat.

### Reagents

- Potassium ferrocyanide trihydrate was purchased from Merck KGaA (Germany). Tris(hydroxymethyl)aminomethane and sodium sulphate were purchased from Sigma-Aldrich (India).
- Ultrapure water (milliQ) was purchased from a milli-q purification system.
- A 0.1 M Tris-nitric solution pH= 7 was prepared with 0.5 M Na2SO4 as inert electrolyte. Electrochemical measurements on the membrane electrodes were performed with a 1 mM potassium ferrocyanide solution in tris-nitric buffer pH=7.

### Membranes

Isophoric polycarbonate membranes with 200 nm nanochannel diameter size were purchased (MF-Millipore Membrane Filters from Merck Life Science S.L.U.

### Exosomes

### Lyophilized exosomes from plasma of healthy donors, Handsa Biomed were purchased (Ref: HBM-PEP-100/5)

### Potentiostat

Electrochemical measurements were carried out with an mStat 400 potentiostat using the software Dropview 8400. Measurements were carried out by cyclic voltammetry (Ei = -0.2 V, Ef = 0.7 V, scan rate v = 50 mV). The measurements were carried out in triplicate.

### Methods

As shown in Fig. 3, firstly, the electrodes were modified by overlaying them with an adhesive-covered protective foil so that only the ceramic region is covered with adhesive (A) and being isolated from the circuit, does not interfere with the electrochemical measurements. In this way, the membrane could be positioned in such a way that it was not damaged during the adhesion process (B).

To remove trapped air bubbles, the same process as in Example 1 with tris nitric solution pH= 7 at 4°C was repeated until no air bubbles were observed.

The measurement of the blank confirmed that no ferrocyanide remained from the previous measurement that could interfere. In this way we obtained a reliable measurement of the membrane electrode, and we can corroborate that all possible changes with respect to the signal are due to the addition of any type of nanoparticle to be blocked.

A measurement of 1 mM ferrocyanide in 0.1 M tris nitric + Na₂SO₄ was performed. We calculated the oxidation intensity before adding the exosome standard Iₐ, then we added 1 µL of different dilutions of the exosome standard solution and let it dry. Finally, a second ferrocyanide measurement was performed to obtain a second oxidation intensity after blocking I_{ab}.

### Results

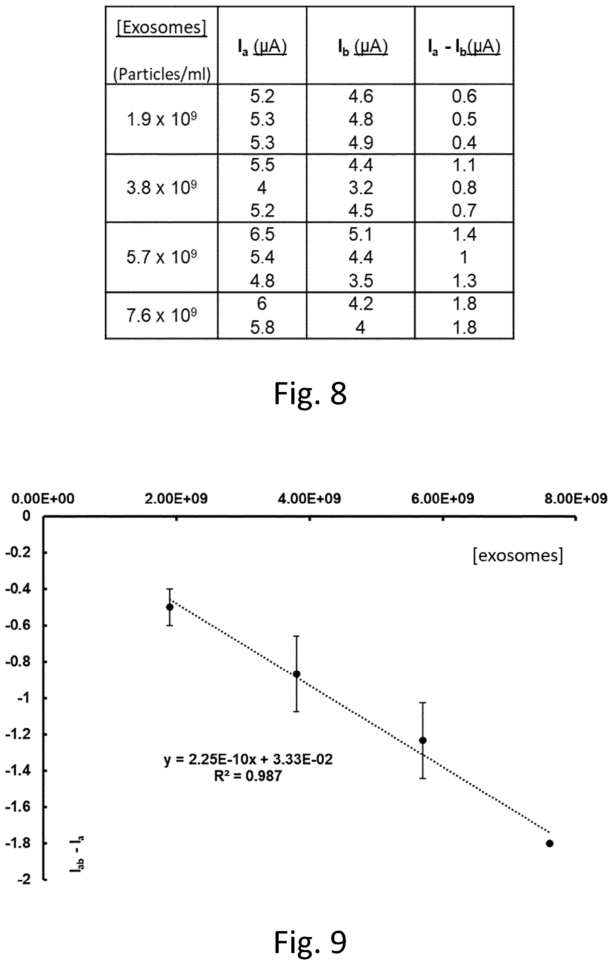

Fig. 8 shows a table with the measurements before (Iₐ) and after (I_{ab}) adding a sample with nanoparticles (exosomes), for different exosomes concentrations between 1.9 × 10⁹ and 7.6 × 10⁹ particles/mL. In all cases, it can be seen that for different measurements the provision of a sample with nanoparticles reduces the Faradaic current (I_{ab-}Iₐ). It can also be noted that for all three measurements, the decrease is similar, and the results are replicable.

Fig. 9 is a calibration curve obtained from the results in Fig. 8, which shows that there is a linear relationship between the reduction in Faradaic current (I_{ab-}Iₐ) and the concentration of nanoparticles in the sample, with high correlation (R = 0.987).

## Claims

1. A system for determining the presence and/or concentration of nanoparticles in a sample (20) comprising a device (100) and a detection reagent (200), wherein the detection reagent (200) comprises an electrochemical probe (210), and wherein the device (100) comprises:
a) an electrochemical sensor (10) **characterized by** comprising at least one working electrode (1) and a second electrode (2);
b) a sample application area (12) in fluid communication with the electrochemical sensor (10);
c) a membrane (3) immobilized on said working electrode (1), the membrane (3) being **characterized by** comprising nanochannels that control the sample (20) access to the working electrode (1); and
d) means (14) for determining the difference in the electrochemical state between the working electrode (1) and the second electrode (2); and
wherein the presence and/or concentration is determined from the change in the difference in the electrochemical state caused by the blockage of the nanochannels by the presence of the nanoparticles in the sample (20).

2. The system according to claim 1 wherein the second electrode is a counter electrode, wherein the difference in the electrochemical state between the working electrode (1) and the counter electrode (2) is the faradaic current between the working electrode (1) and the counter electrode (2) produced by a redox reaction of the electrochemical probe (210).

3. The system according to any one of the previous claims wherein the diameter of the nanochannels of the membrane (3) are comprised between 50 and 1000 nm, preferably between 50 and 100 nm.

4. The system according to any one of the previous claims, wherein the diameter of the nanochannels of the membrane (3) are at least of the size of the nanoparticles whose presence and/or concentration is being determined, preferably at most 25% greater.

5. The system according to claims 2 to 4 when dependent of claim 2, wherein the counter electrode (2) is a silver/silver chloride electrode and wherein the means (14) is a potentiostat (14) configured to provide a potential comprised between - 0.5 V and 1V.

6. The system according to claims 2 to 5 when dependent of claim 2, wherein the means (14) is a potentiostat (14) configured to perform a cyclic or linear voltammetry, preferably wherein the voltage is increased and/or decreased between two values at a rate comprised between 25mV/s and 100 mV/s.

7. The system according to claim 6, wherein the potentiostat (14) is configured to perform a differential pulse voltammetry or square wave voltammetry.

8. The system according to any of the previous claims, wherein the electrochemical probe (210) comprises any of the materials selected from the following list: potassium ferrocyanide, hexamine ruthenium (III) chloride, and ferrocene soluble derivatives such as ferrocene methanol.

9. The system according to any one of the previous claims, wherein the electrochemical sensor (10) is a screen-printed carbon electrode (SPCE).

10. The system according to any one of the previous claims, wherein the working electrode (1) and/or the second electrode (2) comprise any of the materials selected from the following list: carbon, platinum, gold and bismuth.

11. Use of the system according to any one of the claims 1 to 10 to determine the presence and/or concentration of nanoparticles and/or nanovesicles in a food sample (20).

12. Use of the system according to any one of the claims 1 to 10 to determine the presence and/or concentration of nanoparticles and/or nanovesicles in an environmental or cosmetic or pharmaceutical sample (20).

13. Method for determining the presence and/or concentration of nanoparticles in a sample (20), the method comprising the steps:
a) providing a detection reagent (200) according to any one of the claims 1 to 10 onto the sample application area (12) of a device (100) according to any one of the claims 1 to 10;
b) providing the sample (20) onto the sample application area (12) of the device (2);
c) determining the difference in the electrochemical state between the working electrode (1) and the second electrode (2); and
d) determining the presence and/or concentration of the nanoparticles in the sample (20) based on the difference in the electrochemical state determined in step c).

14. Method for determining the presence and/or concentration of nanoparticles in a sample, the method comprising the steps:
a) providing a detection reagent (200) according to any one of the claims 1 to 10 onto the sample application area (12) of a device (100) according to any one of the claims 1 to 10;
b) determining the difference in the electrochemical state between the working electrode (1) and the second electrode (2);
c) providing the sample (20) onto the sample application area (12) of the device (100);
d) determining the difference in the electrochemical state between the working electrode (1) and the second electrode (2); and
e) determining the presence and/or concentration of the nanoparticles in the sample (20) based on the comparison of the difference in the electrochemical state determined in step b) and in step d).

15. Method for determining the concentration of nanoparticles in a sample (20) according to any one of the claims 13 or 14, wherein the second electrode (2) is a counter electrode (2) and wherein the difference in the electrochemical state is the faradaic current between the working electrode (1) and the counter electrode (2).
